Europäisches Patentamt

(19) European Patent Office

Office européen des brevets

(11) Publication number: **0 170 973**
A2

(12) **EUROPEAN PATENT APPLICATION**

(21) Application number: 85109215.5

(22) Date of filing: 23.07.85

(51) Int. Cl.⁴: **C 07 C 29/15,** C 07 C 31/04,
C 07 C 31/08, C 07 C 31/02

(30) Priority: 30.07.84 US 635999

(43) Date of publication of application: 12.02.86
Bulletin 86/7

(84) Designated Contracting States: BE DE FR GB IT NL SE

(71) Applicant: THE DOW CHEMICAL COMPANY, 2030 Dow
Center Abbott Road P.O. Box 1967, Midland,
MI 48640 (US)

(72) Inventor: Conway, Mark M., 949 Walter Road, Sanford
Michigan 48657 (US)
Inventor: Murchison, Craig B., 606 West Meadowbrook
Drive, Midland Michigan 48640 (US)
Inventor: Stevens, Rex R., 4311 Andre, Midland
Michigan 48640 (US)

(74) Representative: Casalonga, Axel et al, BUREAU D.A.
CASALONGA OFFICE JOSSE & PETIT
Baaderstrasse 12-14, D-8000 München 5 (DE)

(54) **Process for adjusting methanol to higher alcohol ratios.**

(57) A process for controlling the ratio of methanol to $C_2-C_5$
alcohols produced in a process for making mixed alcohols by
contacting a hydrogen and carbon monoxide containing feed
with a molybdenum or tungsten containing catalyst under
standard conditions, wherein the improvement concerns the
addition of a sulfur releasing substance in the feed.

EP 0 170 973 A2

ACTORUM AG

PROCESS FOR ADJUSTING METHANOL
TO HIGHER ALCOHOL RATIOS


This invention concerns a process for varying the ratio of methanol to higher alcohols in a Fischer-Tropsch process for making a mixture of alcohols by the addition of a sulfur releasing substance in the feed.

Almost as old as the Fischer-Tropsch process for making hydrocarbons is the Fischer-Tropsch process for making alcohols. The reaction is carried out by passing a mixture of carbon monoxide and hydrogen over a catalyst for the hydrogenation of the carbon monoxide. A typical review article is R. B. Anderson et al., Industrial and Engineering Chemistry, Vol. 44, No. 10, pp. 2418-2424. In this paper the authors list a number of catalysts containing zinc, copper, chromium, manganese, thorium, iron, occasionally promoted with alkali or other materials for making various alcohols. The authors state that ethyl alcohol is a major constituent, the yield of methanol is usually very small and a tentative summary of factors favoring the production of alcohols is high pressure, low temperature, high space velocity, high recycle ratio and carbon monoxide-rich synthesis gas.

Molybdenum is known to be catalytic for the Fischer-Tropsch process and is taught in U.S. Patents 4,151,190 and 4,199,522. The patents describe some of the presently used catalysts but do not teach that the catalyst is useful for making commercially significant quantities of alcohols.

British Patent Publication 2,065,491 discloses a process for making $C_2$ hydrocarbons from $H_2/CO$ using a catalyst comprising a group VB and/or VIB element in combination with an iron group metal as free metals, oxides or sulfides on a porous oxidic support. The authors note that the presence of $H_2S$ alters the activity and selectivity of their process.

U.S. Patent 4,177,202 discloses a process for making hydrocarbons from $H_2/CO$ over a molybdenum and optionally cobalt or vanadium catalyst. Selectivity to ethane is enhanced by presence of hydrogen sulfide in the feed.

U.S. Patent 2,490,488 discloses that molybdenum sulfide methanation catalysts acquire Fischer-Tropsch activity when promoted with an alkaline compound of an alkali metal. The example shows a 30 percent selectivity to $C_3$ and above hydrocarbons and oxygenates. Of this 30 percent, no more than 44 percent boils near or above 65°C, the boiling point of methanol. Accordingly, the maximum possible alcohol selectivity is no more than 13.2 percent (44 percent of 30 percent).

U.S. Patent 2,539,414 describes a Fischer--Tropsch process with molybdenum carbide catalysts which may be used to form oxygenates. At column 3, lines 66-71 the patent teaches that one might get alcohols or hydrocarbons by varying the conditions.

32,676A-F  -2-

EPO Application 81-33,212 (Chemical Abstracts 96:51,800a) discloses a process using rhodium in combination with one or more of a long list of metals which includes molybdenum.

To have a commercially significant alcohol process, one must use a catalyst and conditions which are highly efficient. To be efficient the catalyst must yield a high weight ratio of product per unit weight of catalyst in a given period of time. The catalyst must be stable and active for long periods of time between regenerations. This may be particularly difficult to accomplish when the $H_2/CO$ ratio of the feed gas is low, such as less than 2 to 1. Ideally the catalyst will be sulfur tolerant and will have a high selectivity to a commercial product. Thereby one avoids purification or removal and disposal of by-products and avoids separation into two or more product streams.

For the purposes of this invention, the ratio of $C_1$ to $C_2$+ alcohols means the molar ratio of methanol to $C_2$-$C_5$ alcohols (such as ethanol, propanols, butanols, and pentanols) taken as a whole. The term "alcohols" generally does not include alcohols present as esters or aldehydes. For example, the methanol portion of methyl acetate is not included as methanol.

Excessive methanol is generally considered an unacceptable additive to gasolines. Methanol may decrease drivability, may increase corrosion in the fuel system, and may cause phase separation when used in excessive quantities. These problems may be alleviated by blending methanol with higher alcohols.

Up until now, a method of varying the ratio of $C_1$ to $C_2$ and higher alcohols, short of changing catalysts or distilling the product stream, has not been known. Since distillation adds an additional process step, it is desirable to be able to vary the $C_1$ to $C_2$ and higher alcohols ratio that is produced in the reaction to form the alcohols.

Surprisingly, the process of this invention enables the $C_1$ to $C_2$ and higher alcohol ratio in mixed alcohols to be decreased by the addition of a sulfur releasing substance in the feed. Thus, increasing the concentration of the sulfur releasing compound in the $H_2$/CO feed acts to lower the ratio of $C_1$ to $C_2$ and higher alcohols in the product.

In a process for making mixed alcohols by contacting a hydrogen and carbon monoxide feed with a molybdenum or tungsten containing catalyst in the presence of a promoter, at a temperature of at least 200°C, and run under pressure, the improvement comprises the addition of a sulfur releasing substance in the feed.

The amount of sulfur releasing substance required in the present process is dependent on many factors. Some of the factors are time, pressure, kinetics of the reaction, space velocity, the particular catalyst used, the sulfur releasing substance chosen, and others. The significant end result of the addition of the sulfur releasing substance is obtaining at least a 10 percent by weight decrease in the ratio of methanol to the $C_2$-$C_5$ alcohols produced; mainly the ratio of methanol to ethanol drops.

32,676A-F                    -4-

0170973

Under standard commercial process conditions, the amount of sulfur releasing substance added is at least 25 ppm, with an upper limit for reactivity of the catalyst at 1000 ppm. The preferred amount of sulfur releasing substance is from 50 to 200 ppm.

The hydrogen and carbon monoxide required for this process can be obtained by methods known in the art. Examples are gasification of hydrocarbonaceous materials such as coal, high specific gravity oils, or natural gas; as a by-product of partial combustion cracking of hydrocarbons; by steam reforming of liquid or gaseous hydrocarbons; through the water-gas shift reaction; or some combination of these. The two components may also be generated separately and combined for the subject reaction. The molar ratio of hydrogen to carbon monoxide in the feed gas which contacts the catalyst ranges generally from about 0.25 to about 100, preferably from about 0.5 to about 5 and most preferably from about 0.7 to about 3.

Generally, the selectivity to alcohols is dependent on the pressure. In the normal operating ranges, the higher the pressure at a given temperature, the more selective the process will be to alcohols. The minimum contemplated pressure is about 500 psig (3.55 MPa). The preferred minimum is about 750 psig (5.27 MPa) with about 1,000 psig (7.00 MPa) being a more preferred minimum. While about 1,500 psig (10.44 MPa) to about 4,000 psig (27.7 MPa) is the most desirable range, higher pressures may be used and are limited primarily by cost of the high pressure vessels, compressors and energy costs needed to carry out the higher pressure reactions. About 10,000 psig (69.0 MPa) is a typical maximum with about 5,000 psig (34.6 MPa) a more preferred maximum. About 3,000 psig (20.8 MPa) is a most preferred maximum pressure for $MoS_2$ catalysts.

The selectivity to alcohols is also a function of temperature and is interrelated with the pressure function. The minimum temperature used is governed by productivity considerations and the fact that at temperatures below about 200°C volatile catalytic metal carbonyls may form. Accordingly, the minimum temperature is generally about 200°C.

For a given catalyst, at a constant pressure, as the temperature increases, the selectivity to alcohols decreases. In other words, at lower pressures one is limited to lower maximum temperatures in order to obtain a given selectivity. For example, a preferred maximum temperature is about 400°C. A more preferred maximum is about 350°C. However, the most preferred range of operation is from about 240°C to about 325°C.

The $H_2$/CO gas hourly space velocity (GHSV) is a measure of the volume of hydrogen plus carbon monoxide gas at standard temperature and pressure passing a given volume of catalyst in an hour's time. This may range from about 100 to about 20,000 $hour^{-1}$ and preferably from about 2,000 to about 5,000 $hour^{-1}$. Selectivity to the alcohols generally increases as the space velocity increases. However, conversion of carbon monoxide decreases as space velocity increases.

Preferably at least a portion of the unconverted hydrogen and carbon monoxide in the effluent gas from the reaction, more preferably after removal of product alcohols, water and carbon dioxide formed and even more preferably any hydrocarbons formed, may be recycled to the reaction. The amount of recycle is expressed as the recycle ratio which is the ratio of moles of gases in the recycle stream to the moles of gases in the fresh feed stream. A recycle ratio of zero is within the scope of the process with at least some recycle preferred. A recycle ratio of at least about one is more preferred and at least about three is most preferred.

In addition, the synthesis should be carried out at as little feed conversion per pass as is compatible with economic constraints related to the separation of the alcohol product from unreacted feed and hydrocarbon gases. Accordingly one would increase the space velocity and recycle ratios to preferably obtain about 15-25 percent conversion per pass.

Under the most preferred conditions, alcohols may be obtained in about an 85 percent $CO_2$ free carbon selectivity. The $CO_2$ free carbon selectivity is defined as 100 times the moles of carbon present in a product fraction divided by the total moles of carbon in all products which are not $CO_2$ or unconverted feed. For example, if one mole of ethanol is found in the alcohol fraction, this is counted as 2 moles of carbon. Carbon dioxide and water are not counted as products in this calculation.

The first component of the catalyst preferably consists of molybdenum or tungsten and mixtures thereof in free or combined form. Molybdenum is most preferred. The molybdenum or tungsten may be generally present as the sulfide. Some of the molybdenum or tungsten may be present in combination with other elements such as oxygen or as oxysulfides. The atomic ratio of sulfur to molybdenum or tungsten may range from about 0.1 to about 3 and preferably from about 1.8 to about 2.3. The molybdenum or tungsten may be present in an amount based on the weight of the total catalyst of at least about two percent, preferably at least about 5 percent, with an upper limit of about 98 percent, and preferably about 30 percent of the total catalyst.

When unsupported molybdenum or tungsten is present, it is present in about stoichiometric quantities in relation to other elements with which it may be combined as a compound. Other materials would also have to be considered with respect to the fraction of catalyst that is the active metal, such as, agglomerating agents, binders, pelleting lubricants, promoters and possible other catalytic materials.

The second component of the catalyst is a promoter which may consist essentially of one or more alkali elements or alkaline earth elements in free or combined form. Alkali elements include lithium, sodium, potassium, rubidium and cesium. Alkaline earth elements include beryllium, magnesium, calcium, strontium and barium. Alkali elements and in particular, cesium and potassium, are preferred. Potassium is most preferred.

The promoter may be present in free or combined form as a metal, oxide, carbonate, hydroxide, sulfide or as a salt or a combination of these. The alkaline promoter is preferably present at a level sufficient to render the supported catalyst or the bulk

32,676A-F                    -8-

catalyst more basic. The promoter is generally present in an amount of at least about 0.05 weight percent as a free element in the finished catalyst. Preferably it is present in an amount of at least about 0.1 percent and most preferably at least 0.5 percent. Large amounts up to about 30 percent of the promoter may be present. Preferably the promoter is present at less than 20 percent.

The promoter may be added as an ingredient to the molybdenum or tungsten component or to the support (described below) or may be part of one of the other components such as sodium or potassium molybdate or as an integral part of the support. For example, carbon supports prepared from coconut shells often contain small amounts of alkali metal oxides or hydroxides or the support may contain a substantial amount of the promoter such as when the support is magnesia.

The third optional component of the catalyst is a support which may assume any physical form such as pellets, granules, beads, extrudates, and others. The supports may be (a) coprecipitated with the active metal species, or (b) the support in powder form may be treated with the active metal species and then used as it is or formed into the aforementioned shapes, or (c) the support may be formed into the aforementioned shapes and then treated with the active catalytic species.

The catalytic species may be dispersed on the support by methods known in the art. Examples include: impregnation from solution followed by conversion to the active species; sulfiding of molybdenum or tungsten species; precipitation of the sulfides in the presence of the support; or intimate physical mixing. One or more of these methods may be used. Preferred methods of placing the molybdenum or tungsten sulfide on a support are impregnation with aqueous ammonium tetrathiomolybdate or tetrathiotungstate followed by decomposition to the sulfide and in situ formation of the sulfide by contacting of soluble molybdenum or tungsten salts and a soluble sulfide in the presence of the support. The former is more preferred.

Placing of the molybdenum or tungsten sulfide on the support is preferably followed by treatment with $H_2$ at elevated temperatures.

The exemplary support materials include: the aluminas, basic oxides, the silicas, carbons, or suitable solid compounds of magnesium, calcium, strontium, barium, scandium, yttrium, lanthanum and the rare earths, titanium, zirconium, hafnium, vanadium, niobium, tantalum, thorium, uranium, and zinc, of which oxides are exemplary compounds. Preferably the supports are neutral or basic or may be rendered neutral or basic by addition of the alkaline promoters. The aluminas include the alpha, gamma, and eta types. The silicas include for example, silica gel, diatomaceous earth, and crystalline silicates.

The carbon supports, which are preferred supports, include activated carbons such as those prepared from coals and coal-like materials, petroleum-derived carbons and animal- and vegetable-derived carbons. Preferably the carbon support will have a surface area of $1-1500 \ m^2/g$, more preferably $10-1000 \ m^2/g$ and most preferably $100-500 \ m^2/g$ as measured by the BET nitrogen test.

Preferably, micropores (<20 $\overset{o}{A}$) are minimized and at least twenty percent of the volume of the pores is comprised of pores having a diameter of from about 20 $\overset{o}{A}$ to about 600 $\overset{o}{A}$. Examples include coconut shell charcoal, coals, petroleum cokes, carbons formed by pyrolyzing materials such as vinylidene chloride polymer beads, coal, petroleum coke, lignite, bones, wood, lignin, nut shells, petroleum residues, charcoals, and others.

Based upon the weight of the total catalyst, the support when present generally comprises at least about 20 percent of the catalyst and generally not more than about 98 percent of the catalyst. Preferably the support comprises at least about 50 weight percent, and most preferably at least about 70 weight percent, of the catalyst.

The preferred form of the catalyst is the agglomerated sulfide. Certain forms of molybdenum sulfide are most preferred. Methods for making molybdenum or tungsten sulfide catalysts are disclosed generally at pages 23-34 of Sulfide Catalysts Their Properties and Applications, O. Weisser and S. Landa, Pergamon Press, New York, 1973.

Agglomerated molybdenum sulfide catalysts may be made by thermal decomposition of ammonium tetrathiomolybdate or other thiomolybdates as disclosed in U.S. Patents 4,243,553 and 4,243,554, from purchased active molybdenum sulfides, or by calcining $MoS_3$. The preferred method of preparing the catalyst is by decomposing ammonium tetrathiomolybdate that is formed by reacting a solution of ammonium heptamolybdate with ammonium sulfide followed by spray drying and calcining to form the molybdenum sulfide. The molybdenum sulfide may also be precipitated directly on to a support, but the unsupported molybdenum sulfide is preferred. Tungsten sulfides may be similarly made. An unsupported catalyst preferably has a surface area of at least 10 m²/g and more preferably more than 20 m²/g as measured by the BET nitrogen surface area.

The alkali(ne earth) promoter may be added to the active catalytic element prior to, during or after the formation of the sulfide by physical mixing or solution impregnation. The active metal sulfide may then be combined with binders (such as bentonite clay), and/or pelleting lubricants (such as Sterotex®) and formed into shapes for use as a catalyst.

The finished catalyst may be used in a fixed bed, moving bed, fluid bed, ebullated bed or a graded bed wherein concentration and/or activity of the catalyst varies from inlet to outlet in similar manner to known catalysts. The catalyst may be used in powdered form or may be formed into shapes with or without a binder.

In the present process the catalysts may be employed individually or in combination with other catalysts or with other previously proposed catalysts and activators. In combination with other conventional catalysts, the catalysts may tend to progressively modify the usual effects in accordance with their individual characteristics so that quantitatively intermediate results may be achieved. In summary, the catalysts of the present invention may be combined, for example, with typical hydrogenation catalysts such as cobalt and nickel.

Under preferred conditions the catalyst is stable for long periods of time and under ideal conditions may be stable and active for as many as 6000 hours or more. Activity and selectivity are preferably substantially retained after 700 hours of operation, more preferably after 2000 hours and most preferably after 4000 hours operation.

The alcohol fraction formed using the aforementioned conditions may contain straight or branched-chain alcohols such as methanol, ethanol, 1-propanol, 2-propanol, 1-butanol, 2-butanol, 2-methyl-1-propanol, 2-methyl-2-propanol and other $C_5$-$C_8$ alcohols.

The product mixture, as formed under preferred reaction conditions, contains only small portions of oxygenated compounds other than alcohols. These other oxygenated compounds may not be deleterious to using the product, for example, in motor fuels.

In all cases, the alcohol fraction is formed in at least about 20 percent $CO_2$ free carbon selectivity. Preferably the alcohol fraction is formed in at least about 30 percent $CO_2$ free carbon selectivity, more preferably greater than about 50 percent and ideally can be greater than about 70 percent. By percent $CO_2$ free carbon selectivity it is meant the percent of carbon in a specific product with respect to the total carbon converted from carbon monoxide to some product other than carbon dioxide. For example, one mole of ethyl alcohol is 2 moles of carbon and would represent 50 carbon mole percent selectivity to ethanol if 4 moles of CO were converted to products other than $CO_2$.

Preferably the co-products formed with the alcohol fraction are primarily gaseous products. That is $C_1$-$C_4$ hydrocarbons. By hydrocarbons, it is meant that heteroatoms such as oxygen, sulfur and nitrogen are not present in the molecule. Preferably $C_5$ and higher hydrocarbons are coproduced at less than about 20 percent $CO_2$ free carbon selectivity, more preferably at less than 10 percent, and most preferably at less than 5 percent. Lower amounts of normally liquid hydrocarbons make the normally liquid alcohols easier to separate from by-products.

Under preferred conditions, the amount of water formed is substantially less than the amount of alcohols formed. Typically there is less than about 20 weight percent and preferably less than about 10 weight percent water based on the quantity of alcohol. This water may be removed by known techniques. If the water content is about 2 weight percent or less based on alcohols, the water may advantageously be removed by absorption on molecular sieves. At higher water contents, one may use a water gas shift drying step, for example, as disclosed in British Patents 2,076,015 and 2,076,423. Use of a sulfur and molybdenum or tungsten tolerant catalyst such as Haldor Topsoe SSK is preferred in the water gas shift drying step.

Generally, selectivity to mixed alcohols may be increased by adjusting various reaction parameters, e.g. by increasing pressure, space velocity, product gas recycle ratio and by decreasing conversion, $H_2$/CO feed ratio and temperature.

By adjusting the quantity of sulfur releasing materials that are fed into the process along with the hydrogen/carbon monoxide feed, one can alter the composition of the mixed alcohols formed. Theoretically, it is thought that the sulfur, in molybdenum or tungsten sulfide catalysts used in the present process, is labile under the reaction conditions. Thus the character of the catalyst changes over time as sulfur is removed from the surface of the catalyst during the course of the reaction. This surface sulfur may be replaced or even increased by the present process addition of sulfur releasing compounds into the feed.

By a sulfur releasing compound, it is meant that under the reaction conditions encountered in the process for converting $H_2$/CO to mixed alcohols, the sulfur releasing compound is converted to yield an active sulfur-containing species. The active sulfur species is unknown, but requires molybdenum or tungsten and sulfur.

The mechanism of the sulfur/catalyst interaction is unknown. However, in view of the time lag between adjustment of the sulfur level in the $H_2$/CO feed and a change in the ratio of $C_1$ to $C_2$ and higher alcohols, it appears that the ratio is dependent on the equilibrium sulfur level on the catalyst surface.

Suitable sulfur releasing compounds are organic sulfur compounds, such as mercaptans and sulfides. Inorganic sulfur compounds may also be used as long as they yield sulfur or sulfide upon decomposition. Typical organic sulfur compounds include the mercaptans such as methyl, ethyl and propyl mercaptans, the sulfides

32,676A-F                    -15-

such as diethylsulfide and thiophene. Typical inorganic sulfur releasing compounds are hydrogen sulfide, carbon disulfide and carbonyl sulfide. Hydrogen sulfide is preferred.

One may advantageously use a stream formed by flash fractionation of the alcohol product of the process as a sulfur releasing compound by recycling that stream back into the $H_2/CO$ feed. Most of the sulfur which is removed from the reactor under reaction conditions can be isolated from the alcohol product by a simple flash fractionation. The sulfur-containing compound is taken overhead as a vapor. An appropriate amount of this overhead vapor can then be recycled to the feed in order to adjust the level of sulfur releasing compound in the feed.

An additional method for adjusting the sulfur level in the feed to the alcohol synthesis is by adjusting sulfur removal efficiency in previous process steps. For example if the $H_2/CO$ mixture is formed from a sulfur containing material such as by partial combustion cracking of a crude oil, the sulfur compounds could be removed in a scrubber or absorber prior to utilization in the process for making alcohols. The efficiency of this removal process may be adjusted to obtain the desired level of the sulfur in the feed to the alcohols process.

If one wishes to maintain the current ratio of $C_1$ to $C_2$ and higher alcohols this can be advantageously done by adjusting the sulfur additon rate to the feed to about match the quantity of sulfur being removed in the crude product stream. If one desires to increase or decrease the $C_1$ to $C_2$ and higher alcohol ratio one may adjust the sulfur level in the feed by lowering and/or raising the addition rate of the sulfur releasing compound.

Variation in the sulfur content of the feed to the previously described process can cause the methanol content of the product alcohols to vary from about 15 to about 85 weight percent with nominally the same catalysts in the reactor. In other words, the weight ratio of $C_1$ to $C_2$ and higher alcohols can be varied from about 0.18:1 to about 5.7:1. This corresponds to a molar ratio of $C_1$ to $C_2$-$C_5$ alcohols of from about 0.3 to about 9. Typically, lower sulfur levels in the feed result in higher methanol content; higher sulfur levels in the feed will result in lower methanol contents.

The following examples are provided to illustrate the present process. Many modifications can be made within the spirit and scope of the present process.

Example 1

A hot solution (50°C-60°C) made by combining 10.0 g of ammonium heptamolybdate tetrahydrate $[(NH_4)_6Mo_7O_{24} \cdot 4H_2O]$, 2.1 g of anhydrous potassium carbonate $[K_2CO_3]$, and 60 cm³ of aqueous 22 percent ammonium sulfide $[(NH_4)_2S]$, is added dropwise over 32 g of 12-20 mesh MBV activated carbon (available from Witco Chemical Co.) until the carbon is saturated. All but 11 cm³ of the solution is used.

After air-drying at room temperature until the carbon no longer appears wet, the carbon is heated in flowing nitrogen at a rise of 2°C/min. to 300°C which

0170973

is held for one hour.  The following day this catalyst is air-dried at 150°C for two hours and then is impregnated in the same manner as before using a solution made by combining 8.3 g of $(NH_4)_6Mo_7O_{24} \cdot 4H_2O$, 1.8 g of $K_2CO_3$ and 50 cm³ of aqueous 22 percent $(NH_4)_2S$.  Seven cubic centimeters of solution are left over after the impregnation.  The drying and heat treating steps are repeated.

A third impregnation is carried out using a solution made by combining 3.6 g of $(NH_4)_6Mo_7O_{24} \cdot 4H_2O$, 0.8 g of $K_2CO_3$ and 30 cm³ of aqueous 22 percent $(NH_4)_2S$.  All of the solution is absorbed.  The drying and heat treating steps above are repeated a third time.

Forty cubic centimeters of this catalyst are loaded into a one-half inch (1.27 cm) tubular reactor.  Mass flow meters are used to meter $H_2$, CO, $H_2S$ and $CO_2$ independently over the catalyst.  A gas compressor is used to control the pressure in the reactor.  A sand bath furnace is used to control the temperature in the reactor.  The products from the reactor pass through gas/liquid separation at room temperature and 50-100 psig (0.45-0.79 MPa) followed by a second gas/liquid separation at ambient pressure at dry ice temperature.  Both gaseous and liquid products are analyzed by gas phase chromatography.

In the following Table I, the results of a continuous run of 1a - 1f are given.

TABLE I

| Example | 1a | 1b | 1c |
|---|---|---|---|
| Temp. (°C) | 268 | 270 | 312 |
| Pressure (psig) | 1500 | 2000 | 2000 |
| (MPa) | 10.44 | 13.89 | 13.89 |
| $H_2$/CO (molar ratio) | 1.04 | 1.07 | 1.07 |
| GHSV ($hr^{-1}$) | 1980 | 3000 | 4200 |
| Time at indicated $H_2S$ level (hours) | 49 | 238 | 17 |
| $H_2S$ added (ppm) | 40 | 0 | 138 |
| Time on stream (hours) | 49 | 312 | 563 |
| CO Conversion (%) | 8.0 | 12.0 | 19.0 |
| Wt. units CO converted per wt units of catalyst per hr | 0.150 | 0.337 | 0.810 |
| $CO_2$ produced[1] (%) | 34.0 | 20.4 | 31.4 |
| Selectivities[2] (%) | | | |
| Gas Phase | | | |
| $CH_4$ | 19.6 | 10.7 | 14.0 |
| $C_2^+$ hydrocarbons | 3.7 | 1.7 | 9.2 |
| Subtotal | 23.3 | 12.4 | 23.2 |
| Liquid Phase | | | |
| Methanol | 36.67 | 63.47 | 33.10 |
| Ethanol | 30.39 | 18.66 | 24.80 |
| Propanols | 8.94 | 3.33 | 12.60 |
| Butanols | 0.96 | 0.53 | 3.80 |
| Pentanols | 0.04 | – | 0.95 |
| Subtotal | 77.00 | 85.99 | 75.25 |
| Molar Ratio $C_1$/$C_2$-$C_5$ alcohols | 1.99 | 6.00 | 1.87 |
| Other oxygenates[3] and hydrocarbons | 0 | 1.61 | 1.55 |
| $H_2O$[4] (wt. %) | 2.1 | 1.6 | 5.2 |

## TABLE I (cont'd)

| Example | 1d | 1e | 1f |
|---|---|---|---|
| Temp. (°C) | 320 | 302 | 296 |
| Pressure (psig) | 2500 | 3000 | 3000 |
| (MPa) | 17.34 | 20.79 | 20.79 |
| $H_2$/CO (molar ratio) | 1.14 | 1.14 | 1.14 |
| GHSV ($hr^{-1}$) | 3348 | 3348 | 2310 |
| Time at indicated $H_2S$ level (hours) | 51 | 104 | 48 |
| $H_2S$ added (ppm) | 138 | 0 | 110 |
| Time on stream (hours) | 614 | 718 | 766 |
| CO Conversion (%) | 21.2 | 26.3 | 17.0 |
| Wt. units CO converted per wt units of catalyst per hr | 0.638 | 0.794 | 0.353 |
| $CO_2$ produced[1] (%) | 35.9 | 24.4 | 29.6 |
| Selectivities[2] (%) | | | |
| Gas Phase | | | |
| $CH_4$ | 16.6 | 12.6 | 13.9 |
| $C_2^+$ hydrocarbons | 13.9 | 4.7 | 4.6 |
| Subtotal | 31.5 | 16.3 | 18.5 |
| Liquid Phase | | | |
| Methanol | 16.1 | 50.8 | 24.7 |
| Ethanol | 30.3 | 22.3 | 36.8 |
| Propanols | 14.4 | 6.6 | 13.1 |
| Butanols | 4.84 | 1.6 | 3.7 |
| Pentanols | 1.41 | 0.1 | 1.0 |
| Subtotal | 67.05 | 81.4 | 79.3 |
| Molar Ratio $C_1$/$C_2$-$C_5$ alcohols | 0.75 | 3.69 | 1.03 |
| Other oxygenates[3] and hydrocarbons | 1.45 | 2.3 | 2.2 |
| $H_2O$[4] (wt. %) | 6.9 | 3.1 | 4.6 |

32,676A-F                    -20-

0170973

TABLE I (cont'd)

Footnotes:

[1]$100 \times$ moles of $CO_2$ formed for each mole of CO converted in the reactor.

[2]Selectivities, except for $CO_2$, are based on carbon mole selectivity on a $CO_2$ free basis.

[3]Assumed a carbon number of 4 for other oxygenates.

[4]Water is calculated as weight percent of the liquid phase.

It can be seen that as the level of sulfur in the feed is raised, the ratio of methanol to higher alcohols is lowered and as the level of sulfur is lowered, the ratio is raised. The ratio of methanol to higher alcohols is affected by, but is not proportional to, the sulfur level in the feed.

Example 2

A solution of $(NH_4)_2MoS_4$ is prepared by mixing a solution of 180 g of $(NH_4)_4Mo_7O_{24} \cdot 4H_2O$ in 400 cm³ of water containing 100 cm³ of concentrated $NH_4OH$ with 1300 cm³ of 22 percent aqueous $(NH_4)_2S$. After stirring at 50°C-60°C for two hours, the $(NH_4)_2MoS_4$ solution is poured into a large shallow dish and allowed to evaporate to dryness overnight. The dry, dark red $(NH_4)_2MoS_4$ is calcined for one hour at 500°C in nitrogen to give black $MoS_2$ which is combined in a mortar and pestle with bentonite clay, $K_2CO_3$ and Sterotex® pelleting lubricant to yield a catalyst containing by weight 66 percent $MoS_2$, 10 percent $K_2CO_3$, 20 percent bentonite clay and 4 percent lubricant. The catalyst is then pelletized into 3.2 mm diameter pellets at 30,000 psi (207 KPa).

The experimental procedure for making alcohols is the same continuous procedure as Example 1 except that 20 cm³ of catalyst is used in a 5/8 inch (1.59 cm) diameter reactor.  The results are reported in Table II.

TABLE II

| Example | 2a | 2b | 2c | 2d | 2e |
|---|---|---|---|---|---|
| Temp. (°C) | 260 | 275 | 282 | 275 | 265 |
| Pressure (psig) | 2000 | 2400 | 2300 | 2550 | 3050 |
| (MPa) | 13.89 | 16.65 | 15.96 | 17.68 | 21.13 |
| $H_2$/CO (molar ratio) | 1.12 | 1.25 | 1.20 | 1.10 | 1.18 |
| GHSV ($hr^{-1}$) | 3150 | 3075 | 3195 | 3390 | 5220 |
| Time at indicated $H_2$S level (hours) | 25 | 52 | 95 | 49 | 70 |
| $H_2$S added (ppm) | 25 | 165 | 160 | 60 | 0 |
| Time on stream (hours) | 102 | 339 | 383 | 431 | 600 |
| CO Conversion (%) | 10.2 | 21.0 | 21.8 | 16.2 | 11.2 |
| Wt. Units CO converted per wt. units of catalyst per hr | 0.158 | 0.302 | 0.334 | 0.276 | 0.281 |
| $CO_2$ produced[1] (%) | 27.0 | 31.6 | 32.8 | 26.4 | 24.5 |
| Selectivities[2] (%) | | | | | |
| Gas Phase | | | | | |
| CH₄ | 14.4 | 20.6 | 20.7 | 15.6 | 11.8 |
| $C_2^+$ hydrocarbons | 0 | 3.0 | 3.7 | 1.9 | 0.6 |
| Subtotal | 14.4 | 23.6 | 24.4 | 17.5 | 12.4 |
| Liquid Phase | | | | | |
| Methanol | 48.9 | 30.7 | 27.4 | 37.0 | 50.5 |
| Ethanol | 25.2 | 30.1 | 31.1 | 27.8 | 25.0 |
| Propanols | 6.8 | 9.0 | 10.1 | 9.2 | 7.2 |
| Butanols | 1.6 | 2.8 | 3.0 | 3.3 | 2.0 |
| Pentanols | 0 | 1.5 | 0.9 | 0.6 | 0 |
| Subtotal | 82.5 | 74.1 | 72.5 | 77.9 | 84.7 |

0170973

TABLE II (cont'd)

| Example | 2a | 2b | 2c | 2d | 2e |
|---|---|---|---|---|---|
| Molar Ratio $C_1/C_2-C_5$ alcohols | 3.20 | 1.61 | 1.38 | 2.07 | 3.28 |
| Other oxygenates[3] and hydrocarbons | 3.1 | 2.3 | 3.1 | 4.6 | 2.9 |
| $H_2O$[4] (wt. %) | 0.78 | 1.95 | 2.1 | 1.4 | 1.4 |

Footnotes:

[1]100 x moles of $CO_2$ formed for each mole of CO converted in the reactor.

[2]Selectivities, except for $CO_2$, are based on carbon mole selectivity on a $CO_2$ free basis.

[3]Assumed a carbon number of 4 for other oxygenates.

[4]Water is calculated as weight percent of the liquid phase.

Again, it is seen that as time progresses, the raising of the sulfur level in the feed decreases the ratio of methanol to $C_2-C_5$ alcohols in the product.

0170973

## CLAIMS

1. In a process for making mixed alcohols by contacting a hydrogen and carbon monoxide feed with a molybdenum or tungsten containing catalyst in the presence of a promoter, at a temperature of at least 200°C, and run under pressure, the improvement comprises the addition of a sulfur releasing substance in the feed.

2. A process of Claim 1 wherein the sulfur releasing substance is an organic sulfur compound or an inorganic sulfur compound which is capable of yielding sulfur or sulfide upon decomposition.

3. A process of Claim 1 and 2 wherein the sulfur releasing substance is a $C_1$-$C_3$ mercaptan, a sulfide or thiophene.

4.   A process according to claims 1-3 wherein the sulfur releasing substance is methyl mercaptan diethylsulfide, hydrogen sulfide, carbon disulfide or carbonyl sulfide.

5.   The process of Claim 1 or 4 wherein the sulfur releasing substance is hydrogen sulfide.

6.   The process according to claims 1-5 wherein the amount of sulfur releasing substance is at least 25 ppm.

7.   The process of Claim 6 wherein the amount of sulfur releasing substance is 50 to 200 ppm.

8.   The process of Claim 1 wherein the ratio of methanol to $C_2$-$C_5$ alcohols is decreased.

9.   The process of Claim 8 wherein the ratio of methanol to $C_2$-$C_5$ alcohols is decreased by at least 10 carbon mole percent.

10.   The process of Claim 9 wherein the ratio of methanol to ethanol is decreased by at least 10 carbon mole percent.